# EUROPÄISCHE PATENTSCHRIFT

(11) **EP 1 971 567 B1**
(45) Veröffentlichungstag und Bekanntmachung des Hinweises auf die Patenterteilung: **27.07.2011**
(21) Anmeldenummer: 06807162.0
(22) Anmeldetag: 11.10.2006
(51) Int. Cl.: C07C 67/54, C07C 69/54

(54) **VERFAHREN ZUR AUFREINIGUNG VON (METH)ACRYLATEN**
METHOD OF PURIFYING (METH)ACRYLATES
PROCEDE DE PURIFICATION DE (METH)ACRYLATE

(30) Priorität: 12.01.2006 DE 102006001771
(43) Veröffentlichungstag der Anmeldung: 24.09.2008
(73) Patentinhaber: Evonik Röhm GmbH, 64293 Darmstadt (DE)
(72) Erfinder: SCHMITT, Bardo, 55252 Mainz (DE); KNEBEL, Joachim, 64665 Alsbach-Hähnlein (DE); GRÄFF, Günther, 64665 Alsbach-Hähnlein (DE)
(86) Internationale Anmeldenummer: PCT/EP2006/067293
(87) Internationale Veröffentlichungsnummer: WO 2007/087903

(56) Entgegenhaltungen:
- EP-A1- 0 736 510
- DE-A1- 10 127 941
- DE-A1-102004 014 684

## Beschreibung

Die Erfindung betrifft ein Verfahren zur Reduzierung des Alkoholgehaltes in (Meth)acrylaten und deren Verwendungen.

(Meth)acrylate haben die verschiedensten Anwendungsbereiche. (Meth)acrylate sind Monomere, die in Polymerisationsreaktionen beispielsweise zu Polymethacrylaten umgesetzt werden können. (Meth)acrylatpolymere können aber auch als Bindemittel oder Additiv in Farben, Lacken, Beschichtungen usw. eingesetzt werden. Auch in der pharmazeutischen Industrie werden (Meth)acrylate in Form ihrer Polymeren beispielsweise zur Beschichtung von Tabletten genutzt. Eine hohe Reinheit ist zumeist sehr vorteilhaft.

In der Literatur findet man viele Wege, (Meth)acrylate zu reinigen. Neben der Destillation werden die Behandlung mit Adsorbentien und die Extraktion mit Lösungsmitteln beschrieben.

In der JP 2003261509 wird eine Umesterung in Gegenwart von Sn-Katalysatoren beschrieben. Zur Katalysatorentfernung wird das Reaktionsgemisch mit saurem lonentauscherharz behandelt.

In der JP 01052747 wird die Herstellung von isocyanathaltigen Methacrylaten beschrieben. Zur Aufreinigung wird destilliert und zur Entfernung von Cl-haltigen Verunreinigungen wird mit einem Molsieb (NaA Zeolith) gearbeitet, Mit diesem Verfahren können jedoch nur hydrolisierbare Verbindungen entfernt werden.

EP-A-0736 510 beschreibt ein Verfahren zur Trennung von Methanol und Methl (Meth)acrylat durch Azeotropdestillation.

JP 2003048866 beschreibt eine Umesterung in Gegenwart von Titanaten und die Behandlung mit Tonerde zur Katalysatorentfernung.

Aufgabe der Erfindung war es (Meth)acrylate in hoher Reinheit und mit hohen Ausbeuten herzustellen.

Die Aufgabe wurde gelöst durch ein Verfahren zur Aufarbeitung von (Meth)acrylaten, dadurch gekennzeichnet, dass eine Isocyanat/KatalysatorMischung zugegeben und anschließend destilliert wird.

Es wurde gefunden, dass das erhaltene Produkt nur geringe Mengen an Alkohol enthält. Mit herkömmlichen Aufreinigungsverfahren konnten Restalkoholgehalte unter 0,1 Gew.-% nicht dargestellt werden. Für viele empfindliche Reaktionen, wie beispielsweise anionische Polymerisationen, ist dies ein zu hoher Restalkoholgehalt. Mit dem erfindungsgemäßen Verfahren können Restalkoholgehalte von < 0,01 Gew.-% erreicht werden.

Überraschend wurde gefunden, dass die Aufarbeitung stabil verläuft. Es werden keine Polymerisate als Verunreinigung gefunden, denn eine Nebenreaktion der Isocyanate mit den phenolischen Stabilisatoren war zu befürchten.

Es wurde gefunden, dass die Reinheiten erhöht werden konnten, in Abhängigkeit vom Edukt teilweise auf 99,9%.

Es wird eine Isocyanat/Katalysator-Mischung verwendet.

Als Isocyanate können alle mono- oder polyfunktionellen Isocyanate verwendet werden. Bevorzugt werden alle gängigen Diisocyanate, wie Toluoldiisocyanat, Hexandiisocyanat, Isophorodiisocyanat, 2,2,4-Trimethylhexamethylendiisocyanat, Diphenylmethandiisocyanat, Dicyclohexylmethandüsocyanat.

Als Katalysatoren können alle bekannten lsocyanataktivatoren verwendet werden. Vorzugsweise werden Amine, besonders bevorzugt Diazabicyclooctan, verwendet. Bevorzugt werden aber auch zinnorganische Verbindungen, besonders bevorzugt Dioctylzinnoxid, Zinndilaurat oder Zinndiethylhexanoat, eingesetzt.

Die Isocyanat/Katalysator-Mischung ist zusammengesetzt aus der berechneten Menge Isocyanat und 0,01 bis 1 % Katalysator, bezogen auf das eingewogene Monomer.

Das Isocyanat wird in äquimolaren Mengen, bevorzugt aber im Überschuss, zur Konzentration der Verunreinigungen, wie Wasser und/oder Alkohol, zugegeben.

Das Mengenverhältnis liegt bei 1:1 bis 10:1, besonders bevorzugt für Diisocyanate bei 1:1 (Isocyanat : Verunreinigung).

Die Aufreinigung erfolgt in zwei Schritten. Zuerst werden die (Meth)acrylate bei 60 bis 90 °C, vorzugsweise bei 80 °C 1 bis 6 Stunden nach der Zugabe von Isocyanaten gerührt.

Anschließend werden die (Meth)acrylate destilliert. Die Destillation erfolgt in Abhängigkeit vom (Meth)acrylat bei Normaldruck oder unter Vakuum. Bevorzugt wird die Destillation bei Bedingungen von 60 bis 140 °C und 0.1 bis 10 mbar durchgeführt.

Die Schreibweise (Meth)acrylat bedeutet hier sowohl Methacrylat, wie z.B. Methylmethacrylat, Ethylmethacrylat usw., als auch Acrylat, wie z.B. Methylacrylat, Ethylacrylat usw., sowie Mischungen aus beiden.

Der besonders niedrige Restalkoholgehalt ermöglicht viele Einsatzgebiete der mit dem vorliegenden Verfahren aufgereinigten (Meth)acrylate. Vorzugsweise können diese (Meth)acrylate in anionische Polymerisationen, der Group Transfer Polymerisation (GTP), ATRP, RAFT und allen Polymerisationstechniken, die empfindlich gegenüber Verunreinigungen sind, verwendet werden.

Die im Folgenden gegebenen Beispiele werden zur besseren Veranschaulichung der vorliegenden Erfindung gegeben, sind jedoch nicht dazu geeignet, die Erfindung auf die hierin offenbarten Merkmale zu beschränken.

### Beispiele

### Vergleichsbeispiel 1 (VB 1):

| | |
|---|---|
| 1600,0 g | Ethylhexylmethacrylat, enthaltend 0,040 mol Wasser und 0,160 mol Ethylhexanol |

Der Ansatz wird eingewogen. Die Monomere sind mit 50 -100 ppm HQME (Hydrochinonmonomethylether) versetzt. Das Produkt wird über eine Kolonne destilliert. Nach einem Vorlauf wird die Hauptfraktion als Methacrylat/Produkt erhalten.

### Vergleichsbeispiel 2 (VB 2):

| | | |
|---|---|---|
| 1600,0 g | Ethylhexylmethacrylat, enthaltend 0,070 mol Wasser und , 0,130 mol Ethylhexanol | |
| 78,9 g | = 0,47 mol | Hexamethylendiisocyanat |

Der Ansatz wird ohne Katalysator eingewogen und das Isocyanat im molaren Überschuss, verglichen mit der Summe aus Restalkohol und Wassergehalt, versetzt. Die Monomere sind mit 50 -100 ppm HQME (Hydrochinonmonomethylether) versetzt. Nach drei Stunden Rühren bei ca.
80°C wird das Produkt über eine Kolonne destilliert. Nach einem Vorlauf wird die Hauptfraktion als Methacrylat/Produkt mit reduziertem Alkoholgehalt erhalten.

### Beispiel 3 (B 3):

| | | |
|---|---|---|
| 1580,0 g | Ethylhexylmethacrylat, enthaltend 0,040 mol Wasser und 0,160 mol Ethylhexanol | |
| 33,6 g | = 0,2 mol | Hexamethylendiisocyanat |
| 1,6 g | Zinn-II-Ethylhexanoat (0,1 % bez. auf Ethylhexylmethacrylat) | |

Der Ansatz wird eingewogen und das Isocyanat im molaren Überschuss, verglichen mit der Summe aus Restalkohol und Wassergehalt, versetzt. Die Monomere sind mit 50 -100 ppm HQME (Hydrochinonmonomethylether) versetzt. Nach drei Stunden Rühren bei ca. 80°C wird das Produkt über eine Kolonne destilliert. Nach einem Vorlauf wird die Hauptfraktion als Methacrylat/Produkt mit minimiertem Alkoholgehalt erhalten.

### Beispiel 4 (B 4):

| | |
|---|---|
| 1600,0 g | Butyldiglykolmethacrylat, enthaltend 0,070 mol Wasser und 0,045 mol Butyldiglykol |
| 19,3 g | = 0,115 mol Hexamethylendiisocyanat |
| 1,6 g | Zinn-II-Ethylhexanoat (0,1 % bez. auf Butyldiglykolmethacrylat) |

Der Ansatz wird eingewogen und das Isocyanat im molaren Überschuss, verglichen mit der Summe aus Restalkohol und Wassergehalt, versetzt. Die Monomere sind mit 50 -100 ppm HQME (Hydrochinonmonomethylether) versetzt. Nach drei Stunden Rühren bei ca. 80°C wird das Produkt über eine Kolonne destilliert. Nach einem Vorlauf wird die Hauptfraktion als Methacrylat/Produkt mit minimiertem Alkoholgehalt erhalten.

Alle weiteren Versuche sind analog berechnet. Ansatzberechnung:

| Vers.-Nr.: | Edukte | Restalkohol [mmol] | Wasser [mmol] | Isocyanat [mmol] |
|---|---|---|---|---|
| VB 1 | Ethylhexylmethacrylat | 160 | 40 | 0 |
| VB 2 | Ethylhexylmethacrylat | 130 | 70 | 470 |
| B 3 | Ethylhexylmethacrylat | 160 | 40 | 200 |
| B 4 | Butyldiglykolmethacrylat | 45 | 70 | 115 |
| B 5 | Benzylmethacrylat | 160 | 150 | 310 |
| B 6 | Ethyltriglykolmethacrylat | 210 | 80 | 290 |
| B 7 | Methyltriglykolmethacrylat | 315 | 60 | 375 |

### Analytik:

| Beispiel | Monomer | Restalkohol [Gew.-%] | Wasser [ppm] | Reinheit [Gew.-%] |
|---|---|---|---|---|
| Edukt VB 1 | Ethylhexylmethacrylat | 0,13 | 60 | 99,3 |
| Produkt VB 1 | Ethylhexylmethacrylat | 0,13 | 60 | 99,6 |
| Edukt VB 2 | Ethylhexylmethacrylat | 0,10 | 70 | 99,5 |
| Produkt VB 2 | Ethylhexylmethacrylat | 0,10 | 60 | 99,6 |
| Edukt B 3 | Ethylhexylmethacrylat | 0,13 | 40 | 99,0 |
| Produkt B 3 | Ethylhexylmethacrylat | < 0,01 | 40 | 99,9 |
| Edukt B 4 | Butyldiglykolmethacrylat | 0,46 | 790 | 97,7 |
| Produkt B 4 | Butyldiglykolmethacrylat | < 0,01 | < 10 | 99,2 |
| Edukt B 5 | Benzylmethacrylat | 0,14 | 170 | 99,2 |
| Produkt B 5 | Benzylmethacrylat | < 0,01 | < 10 | 99,7 |
| Edukt B 6 | Ethyltriglykolmethacrylat | 0,23 | 100 | 94,2 |
| Produkt B 6 | Ethyltriglykolmethacrylat | 0,04 | 100 | 97,4 |
| Edukt B 7 | Methyltriglykolmethacrylat | 0,36 | 100 | 97,9 |
| Produkt B 7 | Methyltriglykolmethacrylat | 0,03 | 100 | 99,1 |

| | | | | |
|---|---|---|---|---|
| Vergleichsbeispiel 1 zeigt, dass eine normale Kolonnendestillation nicht zu einer verbesserten Produktqualität hinsichtlich des Restalkoholgehalts führt. Vergleichsbeispiel 2 zeigt, dass eine Behandlung des Ausgangsmaterials mit Isocyanat ohne den üblichen Isocyanat-Katalysator mit anschließender Kolonnendestillation ebenfalls zu keiner Produktverbesserung führt. | | | | |

Beispiele 3 bis 7 zeigen, dass eine erfindungsgemäße Aufarbeitung der (Meth)acrylate zu einer Minimerung des Restalkoholgehalts und damit zu einer Optimierung der Produktqualität führen.

## Patentansprüche

1. Verfahren zur Aufreinigung von (Meth)acrylaten, **dadurch gekennzeichnet, dass** eine lsocyanavKatalysator-Mischung zugegeben und anschließend destilliert wird.

2. Verfahren zur Aufreinigung von (Meth)acrylaten nach Anspruch 1, **dadurch gekennzeichnet, dass** mono- oder polyfunktionelle Isocyanate verwendet werden.

3. Verfahren zur Aufreinigung von (Meth)acrylaten nach Anspruch 1, **dadurch gekennzeichnet, dass** Isocyanataktivatoren oder zinnorganische Verbindungen verwendet werden.

4. Verfahren zur Aufreinigung von (Meth)acrylaten nach Anspruch 3, **dadurch gekennzeichnet, dass** Amine oder Dioctylzinnoxid, Zinndilaurat oder Zinndiethylhexanoat verwendet werden.

5. Verfahren zur Aufreinigung von (Meth)acrylaten nach Anspruch 1, **dadurch gekennzeichnet, dass** das Isocyanat im äquimolaren Verhältnis oder im Überschuss zur Konzentration der Verunreinigung zugegeben wird.

6. Verfahren zur Aufreinigung von (Meth)acrylaten nach Anspruch 5, **dadurch gekennzeichnet, dass** das Isocyanat im Verhältnis von 1:1 bis 10:1 eingesetzt wird.

## Claims

1. Process for purifying (meth)acrylates, **characterized in that** an isocyanate/catalyst mixture is added and then the mixture is distilled.

2. Process for purifying (meth)acrylates according to Claim 1, **characterized in that** mono- or polyfunctional isocyanates are used.

3. Process for purifying (meth)acrylates according to Claim 1, **characterized in that** isocyanate activators or organotin compounds are used.

4. Process for purifying (meth)acrylates according to Claim 3, **characterized in that** amines of dioctyltin oxide, tin dilaurate or tin diethylhexanoate are used.

5. Process for purifying (meth)acrylates according to Claim 1, **characterized in that** the isocyanate is added in an equimolar ratio or in excess relative to the concentration of the impurity.

6. Process for purifying (meth)acrylates according to Claim 5, **characterized in that** the isocyanate is used in a ratio of 1:1 to 10:1.

## Revendications

1. Procédé pour la purification de (méth)acrylates, **caractérisé en ce qu'**un mélange isocyanate/catalyseur est ajouté et on distille ensuite.

2. Procédé pour la purification de (méth)acrylates selon la revendication 1, **caractérisé en ce qu'**on utilise des isocyanates monofonctionnels ou polyfonctionnels.

3. Procédé pour la purification de (méth)acrylates selon la revendication 1, **caractérisé en ce que** des activateurs d'isocyanate ou des composés organostanniques sont utilisés.

4. Procédé pour la purification de (méth)acrylates selon la revendication 3, **caractérisé en ce qu'**on utilise des amines ou de l'oxyde de dioctylétain, du dilaurate d'étain ou de l'hexanoate de diéthylétain.

5. Procédé pour la purification de (méth)acrylates selon la revendication 1, **caractérisé en ce que** l'isocyanate est ajouté dans un rapport équimolaire ou en excès par rapport à la concentration de la contamination.

6. Procédé pour la purification de (méth)acrylates selon la revendication 5, **caractérisé en ce que** l'isocyanate est utilisé dans un rapport de 1:1 à 10:1.
